# EUROPEAN PATENT SPECIFICATION

(11) **EP 1 476 106 B1**
(45) Date of publication and mention of the grant of the patent: **29.04.2009**
(21) Application number: 02792107.1
(22) Date of filing: 20.12.2002
(51) Int. Cl.: A61F 13/02

(54) **DEVICE FOR TREATING A WOUND IN THE SKIN OF A PATIENT**
VORRICHTUNG ZUM BEHANDELN VON WUNDEN AUF DER HAUTOBERFLÄCHE EINES PATIENTEN
DISPOSITIF ET PROCEDE PERMETTANT DE TRAITER UNE PLAIE CUTANEE

(30) Priority: 22.02.2002 NL 1020049
(43) Date of publication of application: 17.11.2004
(73) Proprietor: Tarsus Participatie Maatschappij, 2111 GG Aerdenhout (NL)
(72) Inventor: ZONDAG, Louis, John, NL-1665 TA Heemskerk (NL); DE ZOETEN, Juan, Pedro, NL-1861 GB Bergen (NL); KREIS, Robert, Walter, NL-1943 MH Beverwijk (NL)
(74) Representative: Vernout, Robert
(86) International application number: PCT/NL2002/000859
(87) International publication number: WO 2003/070135

(56) References cited:
- WO-A-01/93793
- DE-A- 2 520 856
- DE-A- 4 431 918
- GB-A- 1 123 826
- GB-A- 1 549 756
- GB-A- 2 300 808
- US-A- 3 645 264
- US-A- 4 608 041
- US-A- 4 898 160
- US-A- 5 944 017
- US-A- 6 117 164
- US-B1- 6 461 379

## Description

The invention relates to a device for treating a wound in the skin of a patient by exposing the wound to a medium which stimulates the healing process, which device comprises at least one wall which can be connected in an at least substantially fluid-tight manner to skin tissue surrounding the wound so as to form an at least substantially fluid-tight space between the wound and the wall, a fluid inlet for introducing said medium into said space, and a fluid outlet. The invention also relates to a device for treating a wound in the skin of a patient by applying a negative pressure on said wound, which device comprises at least one wall which can be connected in an at least substantially fluid-tight manner to skin tissue surrounding the wound so as to form an at least substantially fluid-tight space between the wound and the wall, and a fluid outlet for removing fluid from said space.

Such a device is known from WO 01/93793. The device that is disclosed therein is very effective for treating burns, decubitus wounds, wounds resulting from diabetes and the like, in which the skin is damaged or has been removed altogether over a relatively large area. The wall of the device consists of a transparent PET cover, which is kept spaced from the wound by a layer of foam which is placed on the skin surrounding the wound. Preferably, a mixture of ozone and oxygen is introduced as a healing medium into the space between the wound and the wall during a treatment. It has appeared that the additional and/or simultaneous introduction of distilled water into the space has a strong stimulating effect on the healing process of wounds. As described in EP 0 620 720 the application of a negative pressure on the wound reduces the bacterial density in the wound, and thereby the healing of the wound is also substantially improved. Also removal of superfluous wound fluid through the fluid outlet , often containing bacteria, is supportive to a faster healing process.

Because a inlet or an outlet opening can be easily provided in the wall of the device, and it can be even advantageous to make several opening provisions across the surface of the device which can be opened by the user depending on his needs, a fluid inlet and an fluid outlet in the pre-use state of the device according to the invention must be also understood as opening provisions or even spaces in the wall where the user can make an opening himself.

An important limitation of the known devices is the maximum dimension of the wound that can be treated in this manner. Although the device of WO 01/93793 is flexible to a certain extend and can be adapted to the dimension of the skin surface to be treated, the PET cover will come into contact with the wounds in the case of larger wounds in spite of the presence of the surrounding layer of foam that acts as a spacer element, which leads to adhesion between the cover and the wound. As a result, the area where said adhesion takes place cannot be reached by the medium and very painful damage to the healing skin can easily occur upon removal of the device. In addition, micro-organisms may form in the area where the adhesion takes place, which microorganisms will delay or even counteract the healing process of the wound.

Consequently, the known devices can only be used with wounds of limited dimension.

The object of the invention is to provide a device for treating a wound in the skin of a patient which does not have any limitations as regards the dimension of the wound, and consequently it is in principle capable of comprising practically the entire skin area of a patient without the above-described this advantageous effects occurring, or in any case to a smaller extent. Another object of the invention is to provide a device which is easy to use, which can be produced in a simple and inexpensive manner, which is comfortable to the patient and/or which has a significant stimulating effect on the healing process.

In order to accomplish that objective, the wall area that is to cover the wound is provided with spacers extending towards the wound, which spacers are intended to rest on the wound so as to keep at least part of the wall spaced from the wound. The spacers prevent the wall from adhering to the wound, so that the healing medium can reach the entire wound, or that alternatively a negative fluid pressure can be applied to the entire wound surface and/or wound fluids can be sucked out of the entire wound area.

Preferably, the spacers are arranged a regular distance apart. Preferably, the spacing between the respective centres of the spacers ranges from 8 to 18 mm, preferably it is about 12 mm. In this way a proper balance is found between preventing the occurrence of adhesion between the wound and the wall on the one hand and limiting the wound area on which the spacers rest on the other hand.

Preferably, the wall is made of a flexible material capable of adapting itself to the shape of a respective part of the body. Preferably, the wall is furthermore at least partially made of a transparent material, preferably PVC, polyethylene or silicones so as to enable inspection of the wound during the treatment. Preferably, the spacers themselves are likewise made of these materials, because these materials generally cause little allergic reaction and exhibit poor-adhesion to the wound.

In one preferred embodiment, the spacers are made up of at least one compartment which can be filled with a fluid. Each compartment is preferably fitted with a valve so as to make it possible to fill the compartment with the fluid under pressure and close it. It is especially preferred for the wall and the compartment to be made up of two layers of a foil whose surfaces are bonded together in part, with the parts of the foil that are not bonded together forming the compartment. In a first preferred embodiment, the compartment comprises substantially parallel, tubular sub-compartments. In this way an air mattress-like yet flexible structure is formed which can be placed over the wound and under which the medium has sufficient freedom to reach all the locations of the wound, or fluid can be sucked from the entire surface area of the wound.

In a second embodiment, the compartment comprises substantially square, rectangular, triangular or circular sub-compartments. This gives the device a greater degree of flexibility, enabling it to adapt itself to the shape of the body. Another advantage of the invention is the fact that the air can flow from one sub-compartment to another upon shifting of the patient's weight, as a result of which the pressure on the wound will remain within certain bounds.

In order to enable treatment of large parts of the body in one go in a manner which is simple and relatively comfortable for the patient, the wall is preferably in the form of at least part of a garment, such as a shirt, a pair of trousers, a sleeve, a trouser-leg, a glove and/or a sock, so as to be able to cover a large part of the body. Said parts can be made in various body sizes.

In order to obtain easy adhesion to the skin and effect a substantially fluid-tight seal, the part of the wall that is to come into contact with the skin tissue is preferably provided with adhesive tape, which preferably has a thickness of 1 - 10 mm, more preferably about 3 mm.

The invention also relates to a system for treating a wound on the skin of a patient by exposing the wound to an ozone mixture, which system comprises a device as described above, as well as a device for feeding the ozone mixture to the fluid inlet and/or discharging the ozone mixture from the fluid outlet. Preferably, the device is adjusted so that, in use, a sub-atmospheric pressure prevails in the space between the wall and the wound, for example as a result of the medium being pumped from the outlet by the device. Said sub-atmospheric pressure causes the spacers to exert a pressure on the wound, which helps possible donor skin to adhere to the underlying skin of the patient. Said sub-atmospheric pressure furthermore causes certain bacteria in the skin that retard the healing process to migrate to the surface, where they can be removed with rinsing water.

The invention also relates to a method for treating a wound in the skin of a patient wherein the wound is exposed to a medium which stimulates the healing process, wherein at least one wall is connected in an at least substantially fluid-tight manner to skin tissue surrounding the wound so as to form an at least substantially fluid-tight space between the wound and the wall, and wherein said medium is introduced into said space through a fluid inlet , and removed from said space through a fluid outlet , and wherein the wall area that covers the wound is provided with spacers extending towards the wound, which spacers rest on the wound keeping at least part of the wall spaced from the wound. In a preffered embodiment of the invention said medium comprises ozon. Other mediums can of course be applied too, such as distilled water for just washing the wound, or drug containing mediums.

Furthermore the invention relates to a method for treating a wound in the skin of a patient by applying a negative pressure on said wound, wherein at least one wall is connected in an at least substantially fluid-tight manner to skin tissue surrounding the wound so as to form an at least substantially fluid-tight space between the wound and the wall, wherein fluid is removed from said space through a fluid outlet , and wherein the wall area that covers the wound is provided with spacers extending towards the wound, which spacers rest on the wound keeping at least part of the wall spaced from the wound.

The invention also relates to a method for treating a wound in the skin of a patient, wherein the above mentioned method wherein the wound is exposed to a medium which stimulates the healing process and the above mentioned method wherein a negative pressure is applied on said wound, are alternatingly applied. Hereby often a unexpectedly high pace of wound healing can be achieved.

The invention will be explained in more detail hereinafter by means of embodiments illustrated in the figures, in which like aspects are indicated by like numerals, and in which:
Figure 1 is a top plan view of a first embodiment of a wound treating device;
Figure 2 is a sectional view along the line II-II of the wound treating device of Figure 1;
Figure 3 shows a detail of the longitudinal sectional view of Figure 2;
Figure 4 is a top plan view of a second embodiment of a wound treating device;
Figure 5 is a front elevation of a third embodiment of a wound treating device; and
Figure 6 is a top plan view of a fourth embodiment of a wound treating device.
Figure 7 is a top plan view of a fifth emodiment of a wound treating device.

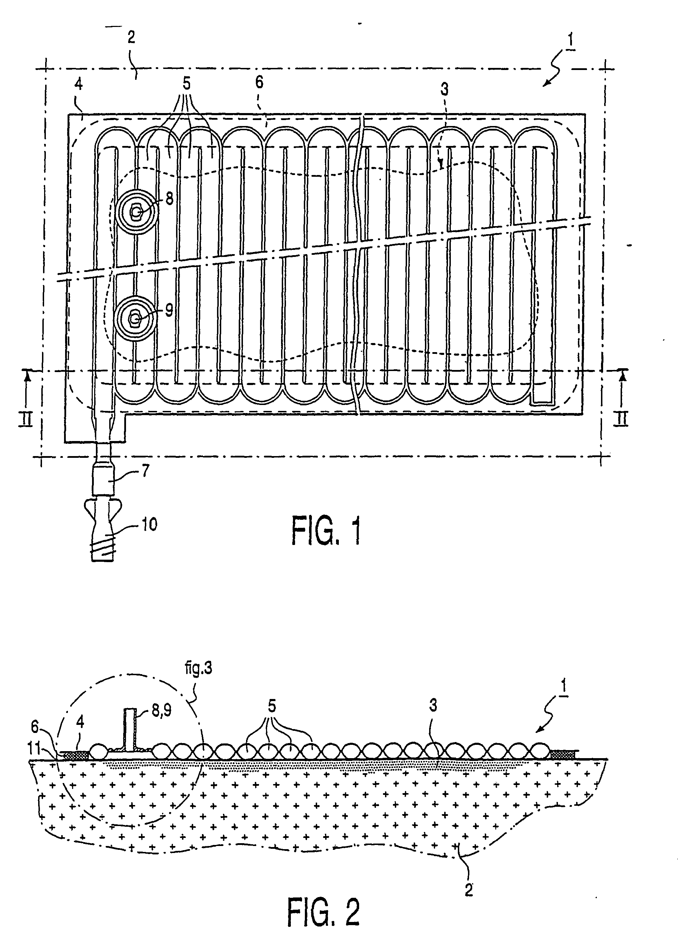

According to Figures 1, 2 and 3, a wound treating device 1 which is placed on the skin 2 over a burn 3 in a patient's skin 2 comprises a transparent wall 4 which is made of two substantially rectangular parts of a flexible PVC foil, the surfaces of two respective sides of which are bonded together in part. The surface of the foil which comes into contact with the wound 2 is provided with a surface structure impressed therein, which helps to prevent the foil from adhering to the wound 2. The bonded-together areas of the PVC foils are configured to form an internal and inflatable continuous tubular compartment which extends into parallel tubular sub-compartments 5 in the wall 4. The wall 4 that is formed in this way is very similar to an inflatable air mattress. Said tubular compartment is provided with a plastic filling aperture 7 with a throttling valve at one end so as to make it possible to fill said "mattress" with air. In an alternative embodiment, the device comprises several separate compartments, which can each be separately filled.

A foam rubber adhesive tape 6 comprising an adhesive layer 11, which has a thickness of about 3 mm, is arranged along the circumference of the wall 4 so as to place the wall 4 over the wound 3 and attach it to the adjoining skin 2 in a substantially liquid-tight manner. The wall 4 is furthermore provided with at least one inlet opening 8 and at least one outlet opening 9 in the form of a plastic inlet and outlet, respectively, which may be identical for that matter. The openings 8, 9 are located between two sub-compartments 5, so that in principle open connections between the surrounding atmosphere and the space 13 between the wound 3 and the wall 4 are formed.

In use, the wound treating device 1 is placed over the wound and the adhesive tape 6 is adhered to the surrounding skin 2 via the adhesive layer 11. The compartment 5 is filled with air and closed by means of the valve 10. A feed line and a discharge line are connected to the inlet opening 8 and the outlet opening 9, respectively. Said lines are connected to a feeding device for distilled water as well as to an ozone generator. The feeding device is so adjusted that a sub-atmospheric pressure will prevail in the space 13 during the treatment. Said sub-atmospheric pressure causes the sub-compartments 5 to be pressed against the wound, resulting-in an improved adhesion of donor skin to the underlying tissue, amongst other things. Clinical tests have shown that treatment for about 20 minutes every day or every other day for a few weeks to a few months, for example, leads to a significantly improved effect as regards the healing of the patient's skin in comparison with the treatment methods that have been usual so far.

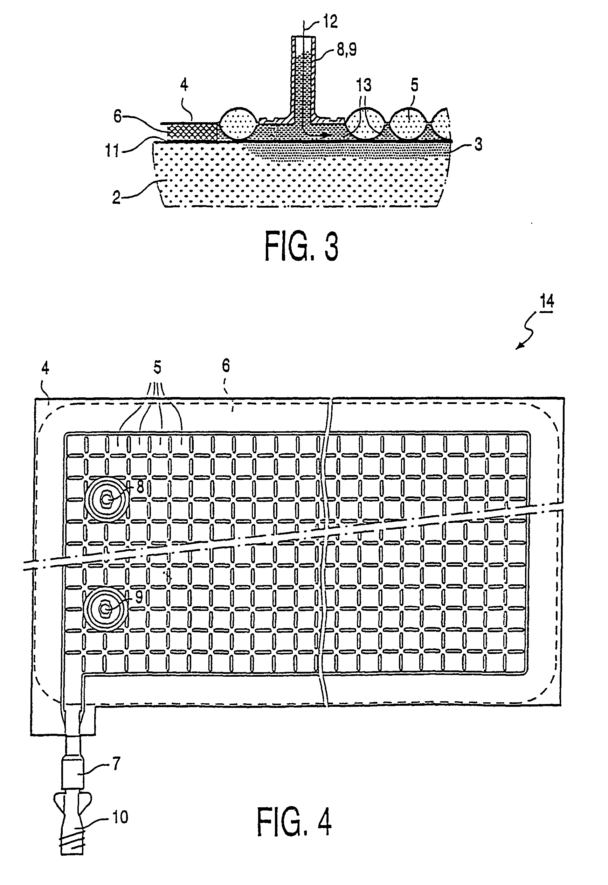

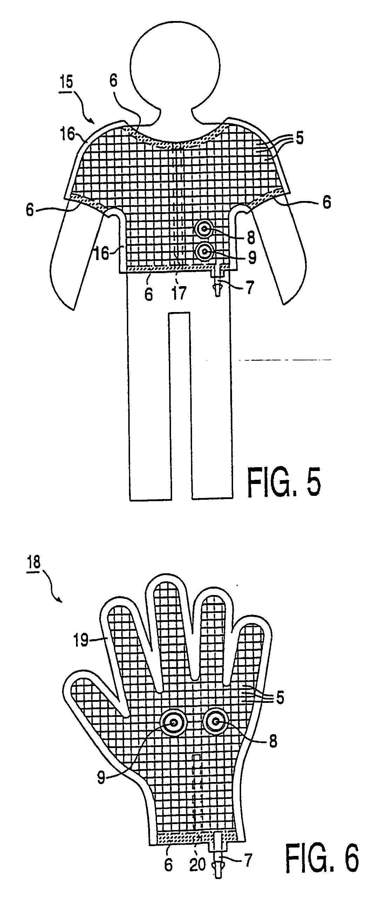

Figure 4 is a view of a second embodiment of a wound treating device 14, the difference with a first embodiment being the fact that the tubular sub-compartments have been substituted for square sub-compartments 5. Said sub-compartments 5 are made by bonding the foils of the wall 4 together along the sides of the sub-compartments 5, with the four corner points forming openings through which air can flow to adjoining sub-compartments. Such a structure is known from flexible containers for producing ice cubes. The advantage of this structure in comparison with the first embodiment is that this device is flexible in two directions, allowing it to adapt more easily to the shape of the body. To that end, various rectangular parts as shown in Figure 1 can be interconnected by means of the adhesive tape, or larger parts configured to conform to the shape of the body can be made. In Figures 5 and 6, two embodiments of wound treating devices 15, 18 in the form of a garment are shown by way of example.

Figure 5 shows a similar wound treating device 15 in the form of a shirt 16 for treating wounds (burns) on the upper body. Although only one inlet opening 8 and one outlet opening 9 are shown, openings 8, 9 may be present at various locations in practice so as to effect a proper distribution of the treating medium over the skin 2. The shirt 16 comprises one or more a fluid-tight closures 17, which enable easy putting on and taking off of the shirt 16. Adhesive tape 6 to be adhered to the skin 2 is arranged along the edges of the skirt 16. Since a sub-atmospheric pressure will prevail in the space 13 under the shirt 16 during use, the shirt 16 will properly remain in position.

Figure 6 shows a wound treating device 18 in the form of a glove 19 for treating wounds (burns) on a person's hand. The glove 19, too, is provided with a fluid-tight closure 20, so that it can be put on and taken off without any difficulty and without causing damage to the skin 2. It will be understood that many variations to the two embodiments that are shown herein are possible for treating wounds at various locations on a person's body.

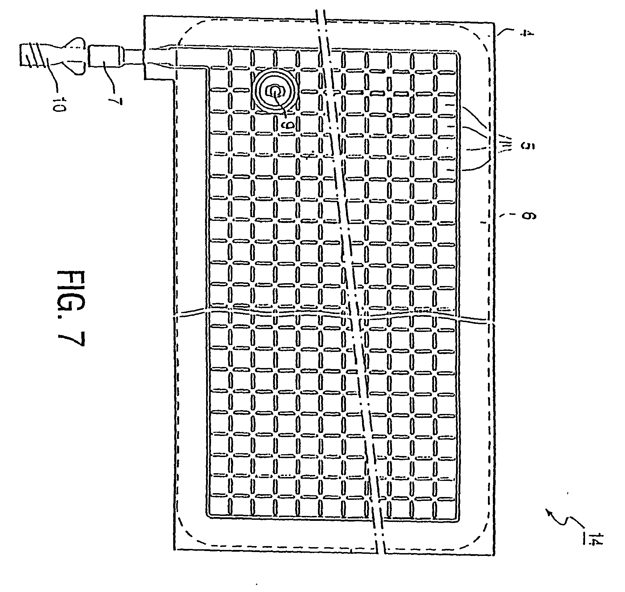

Figure 7 shows a wound treatment device 14 which is very similar to the device of Figure 4, the difference being the fact that there is only a fluid outlet opening 9, and no fluid inlet opening. Of course is possible to close the fluid inlet opening 8 of the device of Figure 4, or to use both, or even more openings to remove fluid from the space 13 between the wound (3) and the wall (4). In this way the device can be used to apply a negative pressure to the wound and remove possible wound fluids. As described in EP 0 620 720 the application of a negative pressure reduces the bacterial density in the wound, and thereby the healing of the wound is substantially improved.

## Claims

1. A device (1, 14, 15, 18) for treating a wound (3) in the skin (2) of a patient by applying a negative pressure on said wound or by exposing the wound to a medium which stimulates the healing process, which device comprises at least one wall (4), means for connecting the circumference of the wall in an at least substantially fluid tight manner to skin tissue surrounding the wound (3) so as to form an at least substantially fluid-tight space (13) between the wound (3) and the wall (4), and a fluid outlet (9) for removing fluid from said space (13), wherein the device is at one side provided with spacers (5) for keeping at least part of the wall (4) spaced from the wound (3), **characterized in that**, the spacers (5) are provided in an area intended to cover the wound (3), which area is located inside and at a distance from the circumference of the wall, and **in that** the spacers are made up of at least one inflatable compartment (5) which can be filled with a fluid, wherein the compartment (5) is fitted with a valve so as to make it possible to fill the compartment (5) with the fluid under pressure and close it.

2. A device (1, 14, 15, 18) according to claim 1, further comprising a fluid inlet (8) for introducing a medium which stimulates the healing process into said space (13).

3. A device (1, 14, 15, 18) according to claim 1 or 2, **characterized in that** the spacers (5) are arranged a regular distance apart.

4. A device (1, 14, 15, 18) according to claim 1, 2 or 3, **characterized in that** the spacing between the respective centres of the spacers (5) ranges from 8 to 18 mm, preferably it is about 12 mm.

5. A device (1, 14, 15, 18) according to any one of the preceding claims 1 - 4, **characterized in that** the wall (4) is made of a flexible material capable of adapting itself to the shape of a respective part of the body.

6. A device (1, 14, 15, 18) according to any one of the preceding claims 1 - 5, **characterized in that** the wall (4) is at least partially made of a transparent material, preferably PVC, polyethylene or silicones so as to enable inspection of the wound (3).

7. A device (1, 14, 15, 18) according any one of the preceding claims 1 - 6, **characterized in that** each compartment (5) is fitted with a valve.

8. A device (1, 14, 15, 18) according to any one of the preceding claims 1 - 7, **characterized in that** the wall (4) and the compartment (5) are made up of two layers of a sheet material whose surfaces are bonded together in part, with the parts of the sheet material that are not bonded together forming the compartment (5).

9. A device (1, 14, 15, 18) according to any one of the preceding claims 1 - 8, **characterized in that** the compartment comprises substantially parallel, tubular sub-compartments (5).

10. A device (1, 14, 15, 18) according to any one of the preceding claims 1 - 9, **characterized in that** the compartment comprises substantially square, rectangular, triangular or circular sub-compartments (5).

11. A device (1, 14, 15, 18) according to any one of the preceding claims 1 - 10, **characterized in that** the spacers (5) are made of PVC, polyurethane or silicones.

12. A device (1, 14, 15, 18) according to any one of the preceding claims 1 - 11, **characterized in that** the wall (4) is preferably in the form of at least part of a garment, such as a shirt (16), a pair of trousers, a sleeve, a trouser-leg, a glove (19) and/or a sock, so as to be able to cover a large part of the body.

13. A device (1, 14, 15, 18) according to any one of the preceding claims 1 - 12, **characterized in that** said connection means comprises adhesive tape (6).

14. A device (1, 14, 15, 18) according to claim 13, **characterized in that** said adhesive tape (6) has a thickness of 1 - 10 mm, preferably about 3 mm.

15. A system for treating a wound (3) on the skin (2) of a patient by exposing the wound (3) to an ozone mixture, which system comprises a device (1, 14, 15, 18) according to any one of the preceding claims 1 - 14, as well as a device for feeding the ozone mixture to the fluid inlet (8) and/or discharging the ozone mixture from the fluid outlet (9).

16. A system for treating a wound (3) on the skin (2) of a patient by applying a negative pressure to said wound (3), which system comprises a device (1, 14, 15, 18) according to any one of the preceding claims 1 - 14, as well as a device for discharging fluid from the fluid outlet (9).

17. A system according to claim 15 or 16, **characterized in that** the device is adjusted so that, in use, a sub-atmospheric pressure prevails in the space (13) between the wall (4) and the wound (3).

## Patentansprüche

1. Vorrichtung (1, 14, 15, 18) zum Behandeln einer Wunde (3) in der Haut (2) eines Patienten durch Anlegen eines Unterdrucks an die Wunde oder durch Einwirken eines Mediums auf die Wunde, das den Heilungsprozess stimuliert, wobei die Vorrichtung wenigstens eine Wand (4), Mittel zum Verbinden des Umkreises der Wand in einer wenigstens im wesentlichen fluiddichten Weise mit Hautgewebe, das die Wunde (3) umgibt, um einen wenigstens im wesentlichen fluiddichten Raum (13) zwischen der Wunde (3) und der Wand (4) zu bilden, und einen Fluidauslass (9) zum Abziehen von Fluid aus dem Raum (13) umfasst, wobei die Vorrichtung an einer Seite mit Abstandsstücken (5) versehen ist, um wenigstens einen Teil der Wand (4) mit Abstand von der Wunde (3) zu halten, **dadurch gekennzeichnet, dass** die Abstandsstücke (5) in einem Bereich vorgesehen sind, der dazu gedacht ist, die Wunde (3) zu überdecken, wobei der Bereich innerhalb und in einem Abstand vom Umkreis der Wand angeordnet ist, und dass die Abstandsstücke aus wenigstens einer aufblasbaren Kammer (5) bestehen, die mit einem Fluid gefüllt werden kann, wobei die Kammer (5) so mit einem Ventil versehen ist, dass es möglich gemacht wird, die Kammer (5) mit dem Fluid unter Druck zu füllen und sie zu schließen.

2. Vorrichtung (1, 14, 15, 18) nach Anspruch 1, die weiter einen Fluideinlass (8) zum Einführen eines Mediums, das den Heilungsprozess stimuliert, in den Raum (13) umfasst.

3. Vorrichtung (1, 14, 15, 18) nach Anspruch 1 oder 2, **dadurch gekennzeichnet, dass** die Abstandsstücke (5) in einem regelmäßigen Abstand voneinander angeordnet sind.

4. Vorrichtung (1, 14, 15, 18) nach Anspruch 1, 2 oder 3, **dadurch gekennzeichnet, dass** der Abstand zwischen den entsprechenden Mitten der Abstandsstücke (5) von 8 bis 18 mm reicht, er vorzugsweise etwa 12 mm beträgt.

5. Vorrichtung (1, 14, 15, 18) nach einem der vorangehenden Ansprüche 1-4, **dadurch gekennzeichnet, dass** die Wand (4) aus einem flexiblen Material besteht, die sich selbst an die Form eines entsprechenden Teiles des Körpers anpassen kann.

6. Vorrichtung (1, 14, 15, 18) nach einem der vorangehenden Ansprüche 1-5, **dadurch gekennzeichnet, dass** die Wand (4) wenigstens teilweise aus einem durchsichtigen Material besteht, vorzugsweise PVC, Polyethylen oder Silikonen, um eine Inspektion der Wunde (3) zu ermöglichen.

7. Vorrichtung (1, 14, 15, 18) nach einem der vorangehenden Ansprüche 1-6, **dadurch gekennzeichnet, dass** jede Kammer (5) mit einem Ventil versehen ist.

8. Vorrichtung (1, 14, 15, 18) nach einem der vorangehenden Ansprüche 1-7, **dadurch gekennzeichnet, dass** die Wand (4) und die Kammer (5) aus zwei Schichten von Folienmaterial bestehen, deren Oberflächen teilweise miteinander verbunden sind, wobei die Teile des Folienmaterials, die nicht miteinander verbunden sind, die Kammer (5) bilden.

9. Vorrichtung (1, 14, 15, 18) nach einem der vorangehenden Ansprüche 1-8, **dadurch gekennzeichnet, dass** die Kammer im wesentlichen parallele, röhrenförmige Unterkammern (5) umfasst.

10. Vorrichtung (1, 14, 15, 18) nach einem der vorangehenden Ansprüche 1-9, **dadurch gekennzeichnet, dass** die Kammer im wesentlichen quadratische, rechteckige, dreieckige oder kreisförmige Unterkammern (5) umfasst.

11. Vorrichtung (1, 14, 15, 18) nach einem der vorangehenden Ansprüche 1 - 10, **dadurch gekennzeichnet, dass** die Abstandsstücke (5) aus PVC, Polyurethan oder Silikonen bestehen.

12. Vorrichtung (1, 14, 15, 18) nach einem der vorangehenden Ansprüche 1-11, **dadurch gekennzeichnet, dass** die Wand (4) vorzugsweise in der Form von wenigstens einem Teil eines Kleidungsstückes vorliegt, wie etwa eines Hemdes (16), einer Hose, eines Ärmels, eines Hosenbeines, eines Handschuhs (19) und/oder eines Strumpfes, um einen großen Teil des Körpers überdecken zu können.

13. Vorrichtung (1, 14, 15, 18) nach einem der vorangehenden Ansprüche 1 - 12, **dadurch gekennzeichnet, dass** das Verbindungsmittel Klebeband (6) umfasst.

14. Vorrichtung (1, 14, 15, 18) nach Anspruch 13, **dadurch gekennzeichnet, dass** das Klebeband (6) eine Dicke von 1-10 mm, vorzugsweise etwa 3 mm besitzt.

15. System zum Behandeln einer Wunde (3) auf der Haut (2) eines Patienten, indem die Wunde (3) einer Ozon-Mischung ausgesetzt wird, wobei das System eine Vorrichtung (1, 15, 14, 18) nach einem der vorangehenden Ansprüche 1-14 umfasst, sowie eine Vorrichtung zum Zuführen der Ozon-Mischung zum Fluideinlass (8) und/oder Ablassen der Ozon-Mischung aus dem Fluidauslass (9).

16. System zum Behandeln einer Wunde (3) auf der Haut (2) eines Patienten, indem ein Unterdruck an die Wunde (3) angelegt wird, wobei das System eine Vorrichtung (1, 14, 15, 18) nach einem der vorangehenden Ansprüche 1-14 umfasst, sowie eine Vorrichtung zum Ablassen von Fluid aus dem Fluidauslass (9).

17. System nach Anspruch 15 oder 16, **dadurch gekennzeichnet, dass** die Vorrichtung so eingestellt ist, dass, im Gebrauch, ein unteratmosphärischer Druck im Raum (13) zwischen der Wand (4) und der Wunde (3) vorherrscht.

## Revendications

1. Dispositif (1, 14, 15, 18) pour traiter une plaie (3) sur la peau (2) d'un patient par application d'une pression négative sur ladite plaie ou par exposition de la plaie à un milieu qui stimule le processus de cicatrisation, dispositif qui comprend au moins une paroi (4), un moyen de connexion de la circonférence de la paroi d'une manière au moins essentiellement hermétique aux fluides au tissu de la peau entourant la plaie (3) de façon à former un espace au moins essentiellement hermétique aux fluides (13) entre la plaie (3) et la paroi (4), et une sortie de fluide (9) pour éliminer le fluide dudit espace (13), dans lequel le dispositif est d'un côté équipé d'espaceurs (5) pour maintenir au moins une partie de la paroi (4) éloignée de la plaie (3), **caractérisé en ce que** les espaceurs (5) sont fournis dans une zone destinée à recouvrir la plaie (3), zone qui est située à l'intérieur et à une certaine distance de la circonférence de la paroi, et **en ce que** les espaceurs sont constitués d'au moins un compartiment gonflable (5) qui peut être rempli avec un fluide, dans lequel le compartiment (5) est équipé d'une vanne de façon à permettre de remplir le compartiment (5) avec le fluide sous pression et à le fermer.

2. Dispositif (1, 14, 15, 18) selon la revendication 1, comprenant en outre une entrée de fluide (8) pour introduire un milieu qui stimule le processus de cicatrisation dans ledit espace (13).

3. Dispositif (1, 14, 15, 18) selon la revendication 1 ou 2, **caractérisé en ce que** les espaceurs (5) sont espacés d'une distance régulière.

4. Dispositif (1, 14, 15, 18) selon la revendication 1, 2 ou 3, **caractérisé en ce que** l'espace entre les centres respectifs des espaceurs (5) s'échelonne de 8 à 18 mm, de préférence elle est d'environ 12 mm.

5. Dispositif (1, 14, 15, 18) selon l'une quelconque des revendications 1 à 4 précédentes, **caractérisé en ce que** la paroi (4) est faite d'un matériau flexible pouvant s'adapter à la forme d'une partie respective du corps.

6. Dispositif (1, 14, 15, 18) selon l'une quelconque des revendications 1 à 5 précédentes, **caractérisé en ce que** la paroi (4) est au moins partiellement faite d'un matériau transparent, de préférence le PVC, le polyéthylène ou des silicones, de façon à permettre une inspection de la plaie (3).

7. Dispositif (1, 14, 15, 18) selon l'une quelconque des revendications 1 à 6 précédentes, **caractérisé en ce que** chaque compartiment (5) est équipé d'une vanne.

8. Dispositif (1, 14, 15, 18) selon l'une quelconque des revendications 1 à 7 précédentes, **caractérisé en ce que** la paroi (4) et le compartiment (5) sont constitués de deux couches d'un matériau en feuille dont les surfaces sont en partie liées les unes aux autres, les parties du matériau en feuille qui ne sont pas liées les unes aux autres formant le compartiment (5).

9. Dispositif (1, 14, 15, 18) selon l'une quelconque des revendications 1 à 8 précédentes, **caractérisé en ce que** le compartiment comprend des sous-compartiments tubulaires essentiellement parallèles (5).

10. Dispositif (1, 14, 15, 18) selon l'une quelconque des revendications 1 à 9 précédentes, **caractérisé en ce que** le compartiment comprend des sous-compartiments essentiellement carrés, rectangulaires, triangulaires ou circulaires (5).

11. Dispositif (1, 14, 15, 18) selon l'une quelconque des revendications 1 à 10 précédentes, **caractérisé en ce que** les espaceurs (5) sont faits de PVC, de polyuréthane ou de silicones.

12. Dispositif (1, 14, 15, 18) selon l'une quelconque des revendications 1 à 11 précédentes, **caractérisé en ce que** la paroi (4) est de préférence sous la forme d'au moins une partie d'un vêtement tel qu'une chemise (16), une paire de pantalon, d'une manche, d'une jambe de pantalon, d'un gant (19) et/ou d'une chaussette, de façon à permettre le recouvrement d'une grande partie du corps.

13. Dispositif (1, 14, 15, 18) selon l'une quelconque des revendications 1 à 12 précédentes, **caractérisé en ce que** ledit moyen de connexion comprend un ruban adhésif (6).

14. Dispositif (1, 14, 15, 18) selon la revendication 13, **caractérisé en ce que** ledit ruban adhésif (6) a une épaisseur de 1 à 10 mm, de préférence d'environ 3 mm.

15. Système pour le traitement d'une plaie (3) sur la peau (2) d'un patient par exposition de la plaie (3) à un mélange d'ozone, système qui comprend un dispositif (1, 14, 15, 18) selon l'une quelconque des revendications 1 à 14 précédentes, ainsi qu'un dispositif pour alimenter en mélange d'ozone l'entrée de fluide (8) et/ou pour décharger le mélange d'ozone par la sortie de fluide (9).

16. Système pour le traitement d'une plaie (3) sur la peau (2) d'un patient par application d'une pression négative sur ladite plaie (3), système qui comprend un dispositif (1, 14, 15, 18) selon l'une quelconque des revendications 1 à 14 précédentes, ainsi qu'un dispositif pour décharger un fluide par la sortie de fluide (9).

17. Système selon la revendication 15 ou 16, **caractérisé en ce que** le dispositif est ajusté de telle sorte que, à l'emploi, une pression inférieure à la pression atmosphérique règne dans l'espace (13) entre la paroi (4) et la plaie (3).
